# EUROPÄISCHE PATENTSCHRIFT

(11) **EP 0 446 675 B1**
(45) Veröffentlichungstag und Bekanntmachung des Hinweises auf die Patenterteilung: **08.12.1993**
(21) Anmeldenummer: 91102443.8
(22) Anmeldetag: 20.02.1991
(51) Int. Cl.: C07C 303/42

(54) **Verfahren zur Herstellung von Na-Salz der Diethylmetanilsäure**
Process for the preparation of sodium 3-diethylaminobenzene-sulfonate
Procédé pour la préparation de 3-diéthylaminobenzène-sulfonate de sodium

(30) Priorität: 28.02.1990 DE 4006226
(43) Veröffentlichungstag der Anmeldung: 18.09.1991
(73) Patentinhaber: BASF Aktiengesellschaft, 67063 Ludwigshafen (DE)
(72) Erfinder: Kolb, Albrecht, Dr., W 6719 Weisenheim (DE); Guenther, Rolf, W-6800 Mannheim 31 (DE); Apel, Wolfram, W-7529 Forst (DE); Rosinger, Manfred, W-6737 Boehl-Iggelheim (DE); Munzinger, Manfred, W-6716 Dirmstein (DE)

(56) Entgegenhaltungen:
- EP-A- 0 293 744
- US-A- 2 488 885
- CHEMICAL ABSTRACTS, Band 34, 1940, Spalte 3246, Zusammenfassung Nr. 4, Columbus, Ohio, US; V.A. IZMAIL'SKII et al.: "Alkylation. VII. Preparation of diethylmetanilic acid and diethyl-m-aminophenol", & J. APPLIED CHEM. (U.S.S.R.) 12, 776-85

## Beschreibung

Die Erfindung betrifft ein Verfahren zur Herstellung von Na-Salz der Diethylmetanilsäure (Wertprodukt) durch Reaktion von Methanilsäure, Ethylchlorid und Natronlauge und Abtrennung des bei der Reaktion entstehenden NaCl aus dem Reaktionsprodukt mittels eines Dekanters.

Die Reaktion verläuft nach folgender Gleichung und angegebenen Reaktionsbedingungen.

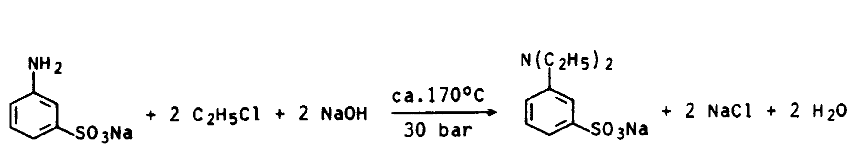

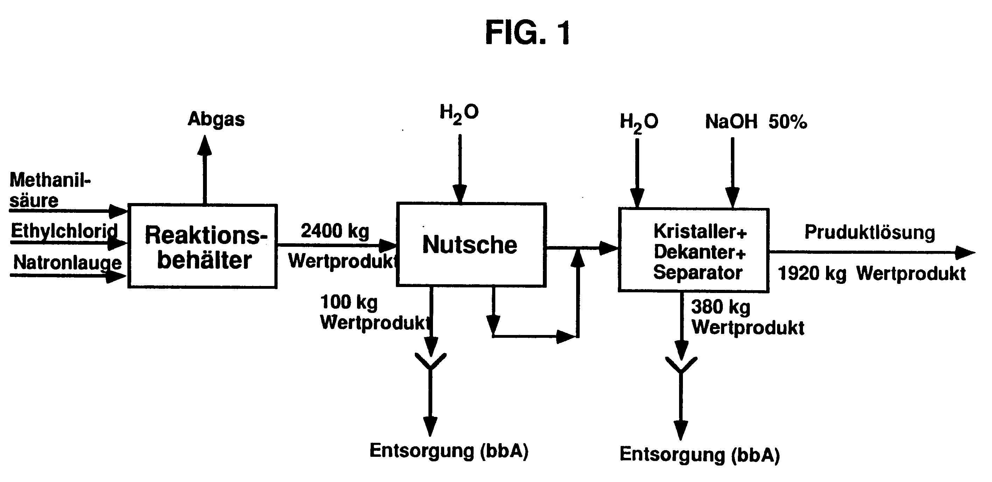

Die Herstellung erfolgt nach dem Stand der Technik wie nachstehend, wobei in einem vereinfachten Verfahrensschema, siehe Figur 1, das Verfahren und die Mengenbilanz bzgl. des Na-Salzes der Diethylmetanilsäure - im weiteren Wertprodukt genannt - aufgezeigt sind.

Während der Reaktion im Reaktionsbehälter - wobei ca. 2400 kg/Charge Wertprodukt entstehen - kristallisiert ein Teil des NaCl aus, dieses wird nach der Reaktion auf einer Nutsche abfiltriert und anschließend mit Wasser gewaschen. Die Waschlösung, die überwiegend Wertprodukt und in geringen Mengen NaCl beinhaltet, wird in die Produktlösung zurückgeleitet, und das NaCl und ca. 100 kg Wertprodukt, nach der Waschung in Wasser gelöst, werden der Entsorgung zugeführt. Die Produktlösung wird vor der anschließenden Kristallisation in einem Kristallisationsbehälter mit Wasser bei 80°C auf 21,5° Be' eingestellt. Nach Zugabe von NaOH-50 %ig zur Reduzierung der Wertprodukt-Löslichkeit wird die Produktlösung unter Rühren auf 20-35°C abgekühlt, dabei kristallisiert das Wertprodukt aus. In nachfolgenden Verfahrensschritten wird mittels eines Dekanters und eines Separators das Kristallisat abgetrennt, hierbei wird als Endprodukt die Produktlösung als Dekanter- und Separatoraustrag mit einem Gehalt von ca. 1920 kg Wertprodukt gewonnen und das Separatorfiltrat mit ca. 380 kg Wertprodukt der Entsorgung zugeführt.

Als Nachteil des Verfahrens erweist sich - wie aus der Mengenbilanz ersichtlich - daß ca. 480 kg Wertprodukt = ca. 20 % der Entsorgung zugeführt werden und damit ein hoher Prozentsatz an Wertprodukt verlorengeht.

Es stellte sich daher die Aufgabe ein Verfahren zur Herstellung des Na-Salzes der Diethylmetaanilsäure zu konzipieren, wobei obiger Verlust des Wertproduktes weitgehend vermieden wird.

Diese Aufgabe wird erfindungsgemäß dadurch gelöst, daß das NaCl-enthaltende Reaktionsprodukt in einem Rührbehälter - ausgerüstet mit Heizmantel und Kondensator - durch thermische Abtrennung von Wasser derart aufkonzentriert wird, daß das Reaktionsprodukt ca. 50 Gew.% Wertprodukt enthält, und wobei anschließend mittels eines Dekanters das Reaktionsprodukt derart getrennt wird, daß der Dekanteraustrag die gesamte Menge NaCl und ca. 2 % Wertprodukt, und das Dekanterfiltrat ca. 98 % Wertprodukt enthält.

Ein Ausführungsbeispiel des erfindungsgemäßen Verfahrens mit den wesentlichen Merkmalen ist in der Zeichnung dargestellt und wird im folgenden näher beschrieben.

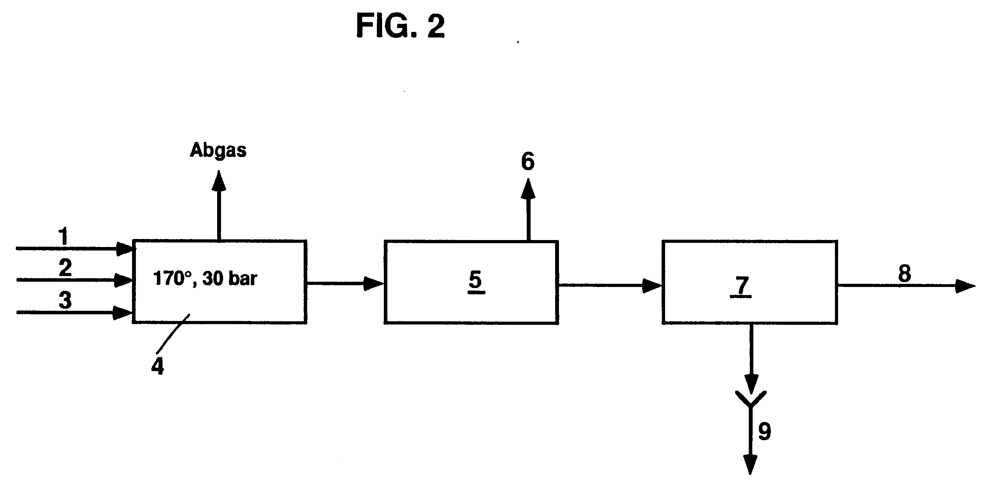

Die Zeichnung - Figur 2 - zeigt ein vereinfachtes Verfahrensschema mit Mengenbilanz und Reaktionsbedingungen.

Nach der Reaktion der Reaktanden - Methanilsäure 1, Ethylchlorid 2 und NaOH 3 - wobei 2400 kg Wertprodukt entstehen, gemäß vorangehender Reaktionsgleichung und vorangehenden Reaktionsbedingungen, in einem Rührbehälter 4, erfolgt in einem weiteren Rührbehälter 5, ausgerüstet mit Heizmantel und Kondensator eine Eindampfung - durch Abtrennung von Wasser 6 - der Produktlösung bei 90 bis 110°C, die bereits auskristallisiertes NaCl enthält. Die Produktlösung wird dabei von ca. 30 Gew.% auf ca. 50 Gew.% Wertprodukt aufkonzentriert, wobei weiteres NaCl auskristallisiert. In einer nächsten Verfahrensstufe wird nun mittels eines Dekanters 7 das kristalline NaCl abgetrennt.

Das Dekanterfiltrat, die aufkonzentrierte Wertproduktlösung 8 - ca. 2352 kg Wertprodukt enthaltend - wird zur Weiterverarbeitung geleitet. Der Dekanteraustrag 9 - im wesentlichen NaCl und ca. 48 kg Wertprodukt enthaltend - wird als behandlungsbedürftiges Abwasser zur Entsorgung geleitet.

Der Verlust an Wertprodukt gemäß dem erfindungsgemäßen Verfahren beträgt ca. 48 kg = 2 %, gegenüber ca. 480 kg = 20 % nach dem Verfahren des Standes der Technik.

## Patentansprüche

1. Verfahren zur Herstellung von Na-Salz der Diethylmetanilsäure (Wertprodukt) durch Reaktion von Methanilsäure, Ethylchlorid und Natronlauge, dadurch gekennzeichnet, daß das NaCl-enthaltende Reaktionsprodukt in einem Rührbehälter - ausgerüstet mit Heizmantel und Kondensator - durch thermische Abtrennung von Wasser derart aufkonzentriert wird, daß das Reaktionsprodukt ca. 50 Gew.% Wertprodukt enthält, und wobei anschließend mittels eines Dekanters das Reaktionsprodukt derart getrennt wird, daß der Dekanteraustrag die gesamte Menge NaCl und ca. 2 % Wertprodukt, und das Dekanterfiltrat ca. 98 % Wertprodukt enthält.

## Claims

1. A process for the preparation of the sodium salt of diethylmetanilic acid (desired product) by reacting metanilic acid, ethyl chloride and sodium hydroxide solution, wherein the NaCl-containing reaction product is concentrated in a stirred container, equipped with a heating jacket and a condenser, by thermal separation of water so that the reaction product contains about 50% by weight of the desired product, the reaction product subsequently being separated by means of a decanter so that the mixture discharged from the decanter contains the total amount of NaCl and about 2% of the desired product and the decanter filtrate contains about 98% of the desired product.

## Revendications

1. Procédé de préparation de sel de sodium de l'acide diéthylmétanilique (produit de valeur) par réaction d'acide méthanilique, chlorure d'éthyle et lessive de soude caustique, caractérisé par le fait que le produit de réaction contenant NaCI est concentré dans un récipient à agitateur - doté d'une enveloppe chauffante et d'un condenseur - par séparation thermique de l'eau, de telle manière que le produit de réaction contienne environ 5 % en poids de produit de valeur et le produit de réaction étant ensuite séparé au moyen d'un décanteur de manière que la décharge du décanteur contienne la quantité totale de NaCI et environ 2 % du produit de valeur et que le filtrat du décanteur contienne environ 98 % du produit de valeur.
